# EUROPÄISCHE PATENTSCHRIFT

(11) **EP 1 038 881 B1**
(45) Veröffentlichungstag und Bekanntmachung des Hinweises auf die Patenterteilung: **13.09.2006**
(21) Anmeldenummer: 99124364.3
(22) Anmeldetag: 07.12.1999
(51) Int. Cl.: C07K 14/675, G01N 33/66

(54) **Verfahren zur Trennung von glykosylierten und nicht-glykosylierten Proteinen**
Process for the separation of glycosylated and non-glycosylated proteins
Procédé de séparation de protéines glycosylées et non-glycosylées

(30) Priorität: 18.12.1998 DE 19858777
(43) Veröffentlichungstag der Anmeldung: 27.09.2000
(73) Patentinhaber: Delta Biotechnology Limited, Nottingham NG7 1FD (GB)
(72) Erfinder: Römisch, Jürgen, Dr., 35041 Marburg (DE); Weisse, Jörg, 35085 Ebsdorfergrund (DE); Stauss, Harald, 35232 Dautphetal (DE); Feussner, Annette, 35043 Marburg (DE)
(74) Vertreter: Meyer-Dulheuer, Karl-Hermann

(56) Entgegenhaltungen:
- US-A- 4 269 605
- BISSE E AND WIELAND H: "Coupling of m-aminophenylboronic acid to s-triazine-activated seaphacryl: use in the affinity chromatography of glycated hemoglobins" J CHROMATOGR, Bd. 575, Nr. 2, März 1992 (1992-03), Seiten 223-228, XP000916537
- SAKAMOTO YASUSHI ET AL: "Structural study of the glycosylated and unglycosylated forms of a genetic variant of human serum albumin (63 Asp fwdarw Asn)." BIOCHIMICA ET BIOPHYSICA ACTA 1995, Bd. 1252, Nr. 2, 1995, Seiten 209-216, XP000916542 ISSN: 0006-3002
- LIN L: "Betaseron" DEV BIOL STAND, Bd. 96, 1998, Seiten 97-104, XP000916548
- KEENAN TW ET AL.: "Tight attachment of fatty acids to proteins associated with milk lipid globule membrane" EUR J CELL BIOL, Bd. 26, Nr. 2, Februar 1982 (1982-02), Seiten 270-276, XP000916520

## Beschreibung

Gegenstand der Erfindung ist ein Verfahren zur chromatographischen Trennung von glykosilierten und nicht-glykosilierten Proteinen.

Es ist bekannt, dass die intrazellulär verlaufende Glykosilierung von Proteinen physiologisch der Stabilisierung der Proteine und ihrer Bindung an zelluläre Rezeptoren dient, die eine Rolle bei Zellaktivierungsprozessen spielen können. Die nichtenzymatische Glykosilierung von Proteinen kann auch extrazellulär vorkommen, wie sie beispielsweise bei Diabetes mellitus beobachtet wird. Im Plasma von zuckerkranken Patienten werden häufig erhöhte Werte an glykosiliertem Albumin nachgewiesen.

Eine besondere Rolle spielen Glykosilierungen bei rekombinanten Proteinen. So hat beispielsweise ein in Hefezellen hergestelltes Protein, das der Aminosäuresequenz des humanen Proteins völlig gleicht, in der Regel ein deutlich unterschiedliches Glykosilierungsmuster, wenn entsprechende Glykosilierungsstellen vorhanden sind. Dies kann zu einer veränderten Halbwertszeit des rekombinanten Proteins, zum Beispiel im Plasma, oder zu einer Bildung von Antikörpern führen, die bei wiederholter Gabe des Proteins allergische Reaktionen provozieren können.

Bei der Expression von rekombinanten Proteinen wird nicht selten beobachtet, dass nur ein Teil des Proteins glykosiliert wird, der nachgewiesen und aus dem Protein entfernt werden muss. Es kann aber auch Fälle geben, in denen die glykosilierte Variante des Proteins das bevorzugte Produkt ist, aus dem das unglykosilierte Protein entfernt werden soll.

Die Trennung der glykosilierten von den nicht-glykosilierten Varianten eines Proteins ist häufig mit den herkömmlichen Methoden wie Chromatographien oder Fällungen nur sehr schwierig zu erreichen. Die N- oder O-glykosidisch an Aminosäuren geknüpften Zucker verändern die physikochemischen Eigenschaften des Proteins, wie den isoelektrischen Punkt, oft nur wenig, so dass zum Beispiel die lonenaustauschchromatographie kaum weiterhilft. In der deutschen Patentanmeldung 198 56 433.3 ist allerdings schon beschrieben worden, dass immobilisierte Lektine, die spezifische Zuckerstrukturen erkennen, bei der Abtrennung von Glykoproteinen hilfreich sein können und in bestimmten Testsystemen als Adsorbens oder Detektionsreagenz eingesetzt werden können. Es besteht aber immer das Risiko des "Blutens" der Matrix, wodurch durch das freigesetzte Lektin zum Beispiel in biologischen Tests, wie in Zellkulturen, erhebliche Störungen auftreten können. Da Lektine eine Immunreaktion hervorrufen können, ist ihre Anwendung zur Herstellung von am Menschen einsetzbaren Arzneimitteln nicht zu empfehlen.

Es wurde nun ein Verfahren zur teilweisen oder vollständigen, chromatographischen Trennung von glykosilierten und nicht-glykosilierten Proteinen gefunden, bei dem man
a) einen auf einer Matrix immobilisierten Triazin-Farbstoff mit einem Gemisch glykosilierter und nicht-glykosilierter Proteine inkubiert,
b) die Matrix dann zur Entfernung der nicht gebundenen Proteine auswäscht und
c) die Proteine durch stufenweise oder kontinuierliche Erhöhung der lonenstärke oder des pH-Wertes eluiert, oder die Bedingungen so gewählt werden, daß das glykosilierte oder das nicht-glykosilierte Protein die Matrix ungebunden durchläuft und das jeweilige gebundene Protein eluiert wird,
wobei in den anfallenden Eluatfraktionen nicht-glykosilierte Proteine und Proteine mit steigendem Glykosilierungsgrad getrennt voneinander aufgefangen werden.

Es ist zwar schon bekannt, dass Farbstoffe der Triazin-Reihe zur Trennung von Proteinen aus Gemischen verwendet werden können (1). Dazu gehören auch plasmatische Proteine wie das Albumin oder aus Hefeextrakten gewonnene spezielle Hefeproteine. Ein Verfahren zur Trennung von glykosilierten und nicht-glykosilierten Bestandteilen eines Proteins, ist jedoch neu und überraschend. Da beispielsweise in Hefen oder anderen eukaryotischen Zellen exprimiertes Albumin partiell glykosiliert ist, besteht die Notwendigkeit, ein Verfahren zur Trennung von glykosilierten Albumin von nicht-glykosilierten Albumin zur Verfügung zu haben. Zudem fallen bei der Gewinnung von rekombinanten Proteinen auch die im Wirtszellextrakt enthaltenen Zellproteine als Verunreinigungen an, deren glykosilierte Bestandteile bei Anwendung des erfindungsgemäßen Verfahrens ebenfalls entfernt werden können. So ist bei Hefeproteinen bekannt, dass insbesondere die glykosilierten Proteine, die häufig mehr oder weniger komplexe Mannosestrukturen aufweisen, bereits in geringen Konzentrationen immunogen im Menschen wirken können. Eine hinreichende Entfernung der glykosilierten Proteine aus dem zur humanen Anwendung vorgesehenen Protein ist also notwendig.

Bei den erfindungsgemäß einzusetzenden Triazin-Farbstoffen handelt es sich insbesondere um Verbindungen aus der Gruppe der Cibacron- oder Procion-Farbstoffe. Auch die Methoden zur kovalenten Koppelung der Triazin-Farbstoffe an Trägermaterialien wie Agarose, vernetztes oder nicht-vernetztes Dextran, Polyacrylamid oder Cellulose sind dem Fachmann bekannt (2, 3, 4). Die Koppelungsverfahren sind meist unkompliziert, schnell und schließen keine toxischen Chemikalien ein. Die Triazin-Farbstoffe zeichnen sich durch eine hohe chemische Stabilität aus, so dass entsprechende Matrices über lange Zeit verwendet und gelagert werden können. Auch gegenüber proteolytischen Aktivitäten und anderen potentiellen Störeinflüssen zeigen solche Farbstoff-Matrices eine hohe Stabilität. Zudem sind die genannten Substanzen vergleichsweise kostengünstig.

In dem erfindungsgemäßen Verfahren wird das Gemisch aus einem oder mehreren Proteinen, das glykosilierte und nicht-glykosilierte Varianten eines oder mehrerer Proteine enthält, mit einer Triazin-Farbstoffmatrix in Kontakt gebracht, die Matrix gewaschen und danach die Proteine eluiert. Zur Elution werden Gradienten steigender Salzkonzentrationen verwendet. Auch pH-Gradienten, fallend oder steigend, eignen sich zur kontinuierlichen oder stufenweisen Elution. Dabei werden die Proteine von ihren glykosilierten Bestandteilen getrennt und fraktioniert.

In einer bevorzugten Ausführung des erfindungsgemäßen Verfahrens wird Albumin bei einem pH-Wert im Bereich zwischen 3,5 und 10 mit einer Farbstoffmatrix in Kontakt gebracht, gewaschen und mittels steigender Salzkonzentration oder steigender pH-Gradienten stufenweise oder kontinuierlich eluiert. Es können auch Bedingungen gewählt werden, unter denen das jeweilige glykosilierte bzw. nicht-glykosilierte Protein die Matrix ungebunden durchläuft, was im Einzelfall nach den Umständen entschieden und z. B. über das pH beeinflußt werden kann. Besonders bevorzugt wird ein Verfahren, bei dem das Albumin mit einem als "Reactive Green 5®" bekannten immobilisierten Farbstoff, vorzugsweise im pH-Bereich von 6 bis 10, besonders bevorzugt in einem Phosphatpuffer, kontaktiert wird. Nach dem Waschen der Matrix wird das von glykosiliertem Protein befreite Albumin durch eine Erhöhung der Salzkonzentration eluiert. Das glykosilierte Protein kann dann durch eine weitere Erhöhung der lonenstärke gewonnen werden.

Besonders bevorzugt ist auch die Verwendung von "Reactive Yellow 3®", das bevorzugt mit einer Albuminlösung im pH-Bereich von 3,5 bis 6,5 in Kontakt gebracht wird. Auch hier kann die separate Elution der glykosilierten und der nicht-glykosilierten Albumine durch Erhöhung der lonenstärke und/oder durch die Erhöhung des pH-Wertes erfolgen.

Außerdem können Zusätze wie Ethylenglykol oder Nukleotide zur Elution verwendet werden. Auch andere Farbstoffe-Matrices wie Cibacron-Blue® können ebenfalls verwendet werden. Die Erfindung wird durch folgendes Beispiel erläutert.

### Beispiel

In transfizierter Bäckerhefe exprimiertes, humaes Albumin kann unter bestimmten Fermentationsbedingungen einen geringen Anteil an mannosyliertem Albumin enthalten. Dieses läßt sich, wie in der deutschen Patentanmeldung 198 56 433.3 beschrieben, quantifizieren. Dabei wird das Lektin Concanavalin A auf einer Mikrotiterplatte immobilisiert und dient als Fänger für glykosilierte, Mannose tragende Moleküle. Nicht gebundenes, unglykosiliertes Albumin wird durch Waschen entfernt und das gebundene Albumin mittels eines markierten, monoklonalen Antikörperfragments bestimmt. Eine Standardkurve dient der Quantifizierung.

Eine so hergestellte Probe wurde durch Gelfiltration in eine Lösung aus 20 mM Na₂HPO₄, pH 7,0, gebracht und anschließend 70 mg Albumin auf eine "Reactive Green 5®"-Agarose-Säule (Firma Sigma; Durchmesser: 1,6 cm, Höhe 10 cm) aufgetragen. Nach Waschen der Säule mit dem vorstehend genannten Puffer wurde ein NaCl-Gradient (0,1 M; Steigung 0,2%/ml) angelegt und Fraktionen zu je 7,5 ml aufgefangen. Die Albuminkonzentration in jeder Probe wurden mittels SEC-HPLC quantifiziert, indem die Peakflächen integriert und an einer Standardkurve der Gehalt abgelesen wurde. Die Gehalte an glykosiliertem Albumin wurden, wie oben beschrieben, bestimmt. Das Verhältnis von glykosiliertem Protein zu Gesamtprotein wurde in Prozent ausgedrückt.

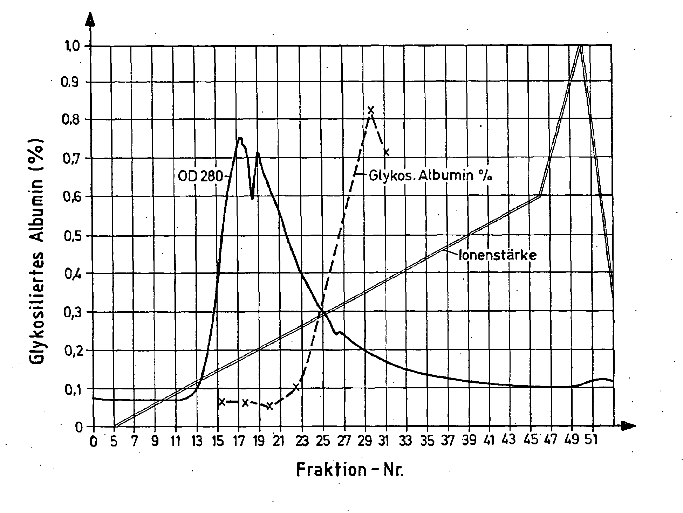

Das Ergebnis ist aus dem als Abb. 1 beigefügten Chromatogramm zu ersehen. Es zeigt den Verlauf der Absorption bei 280 nm, also vor allem das das Fotometer passierende Protein. Daneben ist der kontinuierliche Salzgradient durch Messung der lonenstärke dokumentiert. Nachträglich wurde der errechnete prozentuale Anteil an glykosiliertem Albumin des in der Fraktion nachweisbaren Albumins (gestrichelte Linie) eingetragen. Deutlich zu sehen ist, dass die Fraktionen 13 bis 22 einen gegenüber dem Ausgangsmaterial (0,52%) signifikant erniedrigten Gehalt an glykosiliertem Protein enthielten. Dagegen fanden sich in der Fraktion 30 (ca. 0,8%) und in dem Pool der Fraktionen 50 bis 53 (1,34%) deutlich erhöhte Gehalte des glykosilierten Albumins.

Insgesamt zeigt sich ein deutlicher Trennungseffekt von glykosiliertem und nichtglykosiliertem Albumin, der sich weiter optimieren läßt, zum Beispiel durch Anlegen eines flacheren Salzgradienten.

### Literaturverzeichnis

(1) Dean P.D.G., Watson D.H., J. Chromatogr. 1979; 165:302-319;
(2) Lowe C.R. et al., Int. J. Biochem. 1981, 13:33-40;
(3) Atkinson T. et al., Biochem. Soc. Trans., 1981; 9:290-293;
(4) Easterday R.L. et al., Adv. Exp. Med. Biol. 1974; 42:123-133.

## Patentansprüche

1. Verfahren zur teilweisen oder vollständigen Trennung von glykosilierten und nicht-glykolsilierten Proteinen, **dadurch gekennzeichnet, dass** man
a) einen auf einer Matrix immobilisierten Triazin-Farbstoff mit einem Gemisch glykosilierter und nicht-glykosilierter Proteine inkubiert,
b) die Matrix dann zur Entfernung der nicht gebundenen Proteine auswäscht und
c) die Proteine durch eine stufenweise oder kontinuierliche Erhöhung der Ionenstärke oder eine stufenweise oder kontinuierliche Erhöhung oder Abnahme des pH-Wertes eluiert,
wobei man in den anfallenden Eluatfraktionen nicht-glykosilierte Proteine und Proteine mit steigendem Glykosilierungsgrad getrennt voneinander auffängt.

2. Verfahren nach Anspruch 1, **dadurch gekennzeichnet, dass** die Matrix aus Agarose, vernetztem oder nicht-vernetztem Dextran, Polyacrylamid oder Cellulose besteht.

3. Verfahren nach den Ansprüchen 1 und 2, **dadurch gekennzeichnet, dass** Verbindungen aus der Gruppe der Cibacron- oder Procion-Farbstoffe als Triazin-Farbstoffe eingesetzt werden.

4. Verfahren nach den Ansprüchen 1 bis 3, **dadurch gekennzeichnet, dass** das Proteingemisch mit der Triazin-Farbstoff-Matrix bei pH-Werten zwischen 3,5 und 10 inkubiert wird.

5. Verfahren nach den Ansprüchen 1 bis 4, **dadurch gekennzeichnet, dass** die Mischung der glykosilierten und nicht-glykosilierten Proteine rekombinant in eukaryotischen Zellen oder in transgenen Mikroorganismen hergestellt wird.

6. Verfahren nach Anspruch 5, **dadurch gekennzeichnet, dass** die Mischung der glykosilierte und nicht-glykosilierten Proteine eine Mischung von glykosiliertem und nicht-glykosilierten Albumin ist, das aus Hefezellen erhalten wurde.

## Claims

1. A method for partial or complete separation of glycosylated and non-glycosylated proteins,
**characterized in that**
a) a triazine dye immobilized on a matrix is incubated with a mixture of glycosylated and non-glycosylated proteins,
b) the matrix is then washed out to remove the unbound proteins and
c) the proteins are eluted by a stepwise or continuous increase in the ionic strength or by a stepwise or continuous increase or decrease in pH, whereby non-glycosylated proteins and proteins having increasing degrees of glycosylation are collected separately from one another in the resulting eluate fractions.

2. The method according to Claim 1,
**characterized in that**
the matrix comprises agarose, crosslinked or non-crosslinked dextran, polyacrylamide or cellulose.

3. The method according to Claims 1 and 2,
**characterized in that**
compounds from the group of Cibacron or Procion dyes are used as triazine dyes.

4. The method according to Claims 1 through 3,
**characterized in that**
the protein mixture is incubated with the triazine dye matrix at a pH between 3.5 and 10.

5. The method according to Claims 1 through 4,
**characterized in that**
the mixture of glycosylated and non-glycosylated proteins is produced by recombinant technology in eukaryotic cells or in transgenic microorganisms.

6. The method according to Claim 5,
**characterized in that**
the mixture of glycosylated and non-glycosylated proteins is a mixture of glycosylated and non-glycosylated albumin obtained from yeast cells.

## Revendications

1. Procédé de séparation partielle ou totale de protéines glycolisées et non glycolisées, **caractérisé en ce que**
a) on fait incuber du colorant de triazine immobilisé sur une matrice avec un mélange de protéines glycolisées et non glycolisées,
b) on lave ensuite la matrice pour éliminer les protéines non liées,
c) on élue les protéines par augmentation graduelle ou continue de la force ionique ou augmentation ou réduction graduelle ou continue de la valeur de pH,
tandis qu'on récupère séparément les unes des autres, dans les fractions d'éluat produites, les protéines non glycolisées et les protéines ayant un degré croissant de glycolisation.

2. Procédé selon la revendication 1, **caractérisé en ce que** la matrice est composée d'agarose, de dextrane réticulé ou non réticulé, de polyacrylamide ou de cellulose.

3. Procédé selon les revendications 1 et 2, **caractérisé en ce qu'**on utilise des composés du groupe des colorants de cibacron ou procion comme colorants de triazine.

4. Procédé selon les revendications 1 à 3, **caractérisé en ce que** le mélange de protéines à la matrice de colorant de triazine incube avec des valeurs de pH allant de 3,5 à 10.

5. Procédé selon les revendications 1 à 4, **caractérisé en ce que** le mélange de protéines glycolisées et non glycolisés est préparé par recombinaison dans des cellules eucharyotiques ou des microorganismes transgéniques.

6. Procédé selon la revendication 5, **caractérisé en ce que** le mélange de protéines glycolisées et non glycolisés est un mélange d'albumine glycolisée et non glycolisée ayant été obtenue à partir de cellules de levure.
